# EUROPEAN PATENT APPLICATION

(11) **EP 1 547 606 A1**
(43) Date of publication of application: **29.06.2005**
(21) Application number: 03766691.4
(22) Date of filing: 01.08.2003
(51) Int. Cl.: A61K 35/16, A61P 43/00, A61P 19/08, A61P 17/00, A61P 25/00, A61M 1/02, A61L 27/54

(54) **METHOD OF PREPARING PLATELET RICH PLASMA**

(30) Priority: 02.08.2002 JP 2002226277
(71) Applicant: Sumida, Emi, Tokyo 113-0023 (JP); Toray Industries, Inc., Tokyo 103-8666 (JP)
(72) Inventor: SUMIDA, Emi, Bunkyo-ku, Tokyo 113-0023 (JP); KASUGAI, Shohei, Yokohama-shi, Kanagawa 236-0011 (JP); AKIYOSHI, Kazunari, Chiyoda-ku, Tokyo 102-0081 (JP); IWASAKI, Yasuhiko, Tama-shi, Tokyo 206-0013 (JP)
(74) Representative: Krauss, Jan B., Dr.
(86) International application number: PCT/JP2003/009795
(87) International publication number: WO 2004/012750

(57) **Abstract**

An object of this invention is to provide platelet-rich plasma having high activity easily and at low cost. The object is achieved by a method for preparing platelet-rich plasma comprising a step of adding a water-soluble polymer compound to whole blood obtained by blood collection. For example, by adding poly-L-glutamic acid to whole blood and allowing the mixture to stand still for a definite period, it is possible to obtain platelet-rich plasma having high activity that contains blood plasma components containing much fibrinogen in addition to platelets in the supernatant and blood cell components including white blood cells. This invention also includes the platelet-rich plasma and the use thereof as well as a kit for preparing platelet-rich plasma.

## Description

The present application claims the benefit of priority from Japanese Patent Application No. 2002-226277, which is incorporated herein by reference.

### Technical Field

The present invention relates to platelet-rich plasma used in the field of medical care and a method for preparing the same.

### Background Art

Currently, an increasing number of engineering and medical advances are being made in the field of lost tissue regeneration.

In recent years, however, a large number of incidents have occurred that have jeopardized safety, because blood products used for medical applications and physiologically active substances produced from animals (in particular, cows) are infected with viruses, prions and the like, and this has now become a serious social issue. As a result, there is an extremely high level of interest in the safety of all types of medical care.

Further, at the sites of routine clinical application, importance is attached not only to safety but also to the fact that procedures are reliable and easy to carry out.

Against the above mentioned background, there has been a major focus on a treatment method wherein a patient's own blood components are used to speed up wound healing, the method progressing from fibrin products which have been recognized for some time to effect as an accelerator of postoperative haemostasis and wound healing. For example, because activated blood platelets secrete substances that induce migration and differentiation of cells in the early stage of wound healing, a method has been attempted that a patient's own blood is previously concentrated to make blood plasma rich in platelets ("platelet-rich plasma", hereunder sometimes referred to as "PRP") prior to surgery, which are then activated to apply the PRP to an operation area in order to accelerate healing. A large number of clinicians have reported that wound healing was accelerated by this method (R. E. Marx et al., Oral Surgery Oral Medicine Oral Pathology, Vol. 85, 638-646, 1998; A. K. Garg et al., The Nippon Dental Review, Vol. 62, No.10, 131-143, 2002). The use of a patient's own blood is not only an extremely safe method, but also one with which definite results can be obtained.

However, a method using a patient's own blood involves drawbacks such as complex procedures, risk, labor, necessity of skilled staff, purchase of expensive equipment, and increases in maintenance expenses and the like.

Platelet-rich plasma used for clinical laboratory tests is obtained by collecting blood from intermediate antebrachial vein, adding 4.5 ml of the whole blood to each plastic tube including 1.5 ml of a 3.1% w/v sodium citrate solution, mixing by inversion, and then centrifuging at 50 g for 15 minutes at 22 °C to collect the supernatant (Rinshou Kensahou Teiyou, 31st edn., p. 400 (in Japanese)). Although differences exist in the amount of blood collected and centrifugation conditions, the collection of PRP for the purpose of accelerating tissue healing is also conducted basically according to this method. Blood platelets have extremely abundant reactivity, and are prone to undergo an agglutination reaction or release reaction in the separation process, leading to loss of the functions thereof. Therefore, in the separation procedures, it is necessary for a person of experience to prepare the plasma while paying particular attention. Further, because a decrease in function over time is noticeable for blood platelets in comparison with other blood cell components, it is necessary for separation to be conducted as quickly as possible after blood collection, and this is also a factor that remarkably lowers usability.

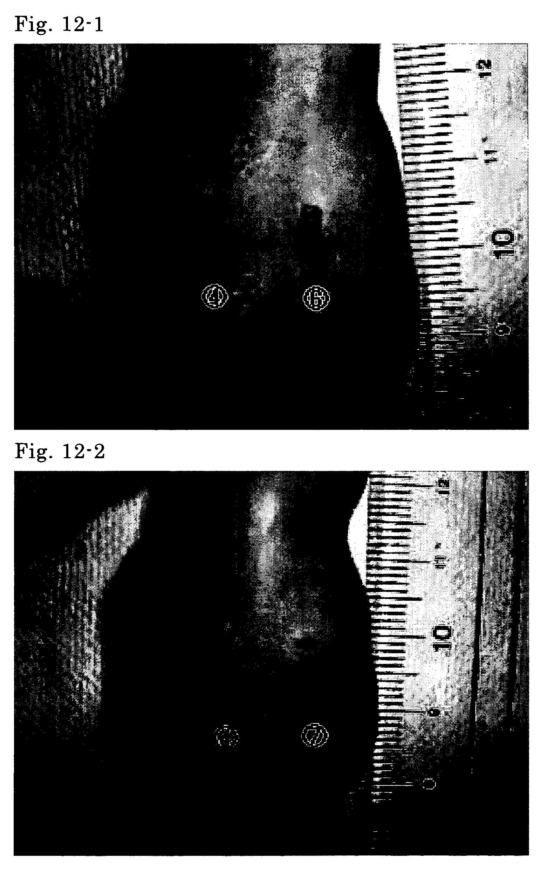

In recent years, a kit for facilitating easy acquisition of PRP used to accelerate tissue healing has been sold commercially. However, the conventional method of acquiring PRP is one that basically uses centrifugation techniques (Hosokawa, T., et al., Dental Outlook, Vol. 100, No.6, p. 1230-1243, 2002), and improvements to the complexities of the above procedures are inadequate therein. Indeed, the complexity of the procedures has been noted not only by the present inventors, and Hosokawa et al. also wrote in the description for Figure 12 on page 1239 of the aforementioned article that "the procedures are somewhat complex." In addition, although separation of red blood cells is adequate in PRP acquired by this centrifugation method, white blood cells included in the whole blood are also separated and thus there are cases in which almost no white blood cells are included in the prepared PRP, and part of the important blood plasma components such as fibrinogen have also been centrifuged.

Examples of the characteristics required for platelet-rich plasma used as an accelerator of postoperative wound healing and haemostasis include that only a small number of red blood cells that cause delays in tissue healing be contained therein, that the plasma includes a large number of blood platelets, that the activity of blood platelets be maintained to a high degree, and that the plasma includes blood components such as white blood cells and fibrinogen in addition to blood platelets. There is a demand for the development of a method for simply and conveniently acquiring platelet-rich plasma with this kind of high activity.

### Disclosure of the Invention

It is an object of the present invention to simply and conveniently acquire platelet-rich plasma with high activity and to provide the same.

As the result of concentrated research, the present inventors discovered that when a water-soluble polymer compound or a pharmacologically acceptable salt thereof is added to blood plasma containing red blood cells of whole blood or the like, not only can platelet-rich plasma be separated quickly and simply, but that the platelet-rich plasma obtained in this manner exhibits extremely high tissue healing activity in comparison with platelet-rich plasma obtained by the conventional method, to thus complete the present invention.

More specifically, this invention comprises the following:
1. A method for preparing platelet-rich plasma, comprising a step of agglutinating and sedimenting red blood cells in a selective acceleratory manner from blood;
2. The method for preparing platelet-rich plasma according to the preceding 1, wherein the blood is whole blood obtained by blood collection;
3. The method for preparing according to the foregoing 1 or 2, wherein the method of agglutinating and sedimenting red blood cells in a selective acceleratory manner comprises a step of adding a water-soluble polymer compound to blood;
4. A method for preparing platelet-rich plasma, comprising a step of adding a water-soluble polymer compound to blood;
5. The method for preparing according to the foregoing 3 or 4, wherein the water-soluble polymer compound is a polymer compound having a molecular weight of 1,000 - 5,000,000;
6. The method for preparing according to the foregoing 3 or 4, wherein the water-soluble polymer compound is added in an amount of 0.0001 - 10 w/v% with respect to a blood volume;
7. The method for preparing according to the foregoing 3 or 4, wherein the water-soluble polymer compound is at least one kind selected from the following compounds:
   1) a polyamino acid comprising amino acids and/or pharmacologically acceptable salts of amino acids,
   2) an acidic polysaccharide and/or pharmacologically acceptable salts thereof, and
   3) a vinyl polymer;
8. The method for preparing according to the foregoing 7, wherein the polyamino acid is at least one kind selected from the group consisting of polyglutamic acid, polyaspartic acid, polyhistidine and polyasparagine;
9. The method for preparing according to the foregoing 7, wherein the amino acids and/or pharmacologically acceptable salts, which are formed polyamino acid, are selected from the group consisting of glutamic acid, aspartic acid, histidine and asparagine, or pharmacologically acceptable salts of these;
10. The method for preparing according to the foregoing 9, wherein at least 20% of the amino acids which the polyamino acid comprises is glutamic acid and/or aspartic acid, or pharmacologically acceptable salts of these;
11. The method for preparing according to the foregoing 9 or 10, wherein the polyamino acid is an acidic polyamino acid;
12. The method for preparing according to the foregoing 7, wherein the acidic polysaccharide and/or pharmacologically acceptable salts thereof is at least one kind selected from the group consisting of dextran derivatives, glycosaminoglycan, cellulose derivatives, chitosan derivatives, galacturonic acid and alginic acid, or pharmacologically acceptable salts of these;
13. The method for preparing according to the foregoing 7, wherein the acidic polysaccharide and/or pharmacologically acceptable salts thereof is hyaluronic acid or a pharmacologically acceptable salt thereof;
14. The method for preparing according to the foregoing 7, wherein the vinyl polymer is at least one kind selected from the compounds including an acidic polymer or a pharmacologically acceptable salt thereof;
15. A platelet-rich plasma prepared by the method according to any one of the foregoing 1 - 14;
16. An accelerator of tissue and/or organ repair, which comprises the platelet-rich plasma according to the preceding 15;
17. An accelerator of tissue and/or organ repair, an additive for bone augmentation in the periphery of a dental implant, an additive for use when transplanting bone or artificial bone to a bone defect site, a wound healing accelerator, an accelerator of tissue repair after therapy or treatment for plastic and/or cosmetic purposes, a therapeutic agent for dermatosis, a therapeutic agent for cutaneous ulcers, an agent for nerve tissue repair and/or an agent for postoperative tissue repair, which comprises the platelet-rich plasma according to the foregoing 15;
18. A therapeutic method or a treatment method for any of the following, which comprises a step of administering the platelet-rich plasma according to the foregoing 15:
   1) bone augmentation in the periphery of a dental implant,
   2) dermatosis,
   3) tissue repair for plastic and/or cosmetic purposes,
   4) repair of a bone defect site,
   5) nerve tissue repair, and
   6) postoperative tissue repair;
19. A reagent or a reagent kit for preparing platelet-rich plasma that comprises at least one kind of the components described below from among water-soluble polymer compounds used for preparing platelet-rich plasma by a method comprising a step of adding a water-soluble polymer compound to blood:
   1) a polyamino acid comprising amino acids and/or pharmacologically acceptable salts of amino acids,
   2) an acidic polysaccharide and/or pharmacologically acceptable salts thereof, and
   3) a vinyl polymer.
20. An instrument for preparing platelet-rich plasma by adding at least one kind of the component of water-soluble polymer compounds described below to blood:
   1) a polyamino acid comprising amino acids and/or pharmacologically acceptable salts of amino acids,
   2) an acidic polysaccharide and/or pharmacologically acceptable salts thereof, and
   3) a vinyl polymer.
21. A kit for preparing platelet-rich plasma, which comprises the reagent or the reagent kit according to the foregoing 19 and the instrument according to the foregoing 20.

### Brief Description of the Drawings

Figure 1 is a schematic diagram of a photograph of peripheral blood obtained using a differential interference microscope (Example 1).
Figure 2 is a schematic diagram of a photograph of supernatant of peripheral blood obtained using a differential interference microscope (Example 1).
Figure 3 is a schematic diagram of an image of supernatant obtained with sample 2 (Example 1).
Figure 4 is a schematic diagram of an image of supernatant of sample 1 in which blood coagulation was observed (Example 1).
Figure 5 is a schematic diagram of an image of sample 4 (Example 1).
Figure 6 is a view showing effects obtained when various kinds of polyamino acids were added to samples and the samples were allowed to stand for 1 hour (Example 4).
Figure 7 is a view showing effects obtained when varying amounts of hyaluronic acid were added to samples and the samples were allowed to stand for 40 minutes (Example 5).
Figure 8 shows effects obtained when various kinds of water-soluble polymers were added to samples and the samples were allowed to stand for 30 minutes (Example 7).
Figure 9 shows effects obtained when various kinds of water-soluble polymers were added to samples and the samples were allowed to stand for 30 minutes (Example 8).
Figure 10 shows a wound healing effect of PRP according to the method of this invention (Experimental Example 4).
Figure 11 shows a wound healing effect of PRP according to the method of this invention on the fourth day after surgery, and the wound healing effect of PRP according to the conventional method on the same day (Experimental Example 5).
Figure 12 shows wound healing effects on the fourth day after surgery for PRP according to the conventional method and for a substance in which white blood cells were added to PRP according to the conventional method (Experimental Example 6).

### Description of Symbols

① Red blood cells
② White blood cells
③ Blood platelets
④ Untreated (control)
⑤ Administration of PRP according to the method of this invention
⑥ Administration of PRP according to the conventional method
⑦ Administration of white blood cells to PRP according to the conventional method

### Best Modes for Carrying Out the Invention

It is desirable that the platelet-rich plasma of this invention comprises not only blood platelets, but also blood plasma components that contain a large amount of fibrinogen, and blood components including white blood cells are further desirably comprised. Blood platelets fulfill an extremely important role when adhering to damaged subendothelial tissue and coagulating to form thrombi and arrest hemorrhage, or when storing and releasing substances that induce migration and differentiation of other cells. Fibrin is a substance that is generated when thrombin acts on fibrinogen in blood plasma, and is therefore a substance that is involved in the final stage of blood coagulation. It is also important as a scaffold for infiltration and cytodifferentiation by a cell to conduct tissue repair. Further, white blood cells have activity that prevents infiltration by bacteria or harmful microorganisms or the like, have immunological functions or bactericidal functions, and promote tissue repair by protecting damaged tissue. It is known that among white blood cells, monocytes/macrophages also play an important role in tissue repair.

Blood platelets lose their function rapidly with stimulation or with passage of time from the time of blood collection. Therefore, it has not been possible to obtain blood platelets that retained a high level of functions by a method other than centrifugation that enabled separation in a short time with little stimulation with respect to blood platelets. Although when whole blood is allowed to stand the red blood cells gradually sediment, the speed thereof is normally extremely slow, and thus it is known that from the other blood than those in a pathological state such as a high inflammatory response, blood plasma that includes blood platelets can not be obtained even when whole blood is allowed to stand for several hours or more.

According to the method for preparing platelet-rich plasma of this invention it is possible to sediment red blood cells from whole blood in a selective and accelerative manner, and to obtain in the supernatant not only blood platelets but also components that are essential for tissue repair, such as white blood cells and fibrinogen, in a state in which they retain a high level of function.

According to this invention, the term "step of agglutinating and sedimenting red blood cells in a selective and accelerative manner" refers to a step of sedimenting 80% or more, preferably 90% or more, of red blood cells included in whole blood by agglutination to obtain platelet-rich plasma comprising at least 15% or more of the total blood volume within 3 hours from the start of treatment, preferably within 2 hours, more preferably within 1 hour, and further preferably within 30 minutes.

Whole blood used for the preparation of platelet-rich plasma of this invention may be acquired according to an ordinary method of collecting blood from a human or a non-human animal. Further, since whole blood acquired by blood collection may coagulate in that state, preferably an anticoagulant is previously added after the blood collection. As the anticoagulant, any substance ordinarily used as an anticoagulant and which does not exhibit toxicity towards living organisms may be used, and a substance that is in general use may be used, for example, sodium citrate, ACD, EDTA, heparin, low molecular weight heparin, futhan, hirudine or argatroban.

The volume of treated blood for preparing the platelet-rich plasma of this invention is not particularly limited and will differ according to the intended use, and a suitable volume can be selected according to the purpose of use and usage amount. The method of this invention can, for example, be applied to blood obtained by collection of a patient's own blood, and can also be applied to blood obtained by blood donation or the like. Since this invention is a method that simply and conveniently obtains blood platelets having a high level of activity, the method can also be used for the preparation of platelet products.

(Water-soluble polymer compound) - In order to prepare the platelet-rich plasma of this invention from blood obtained by blood collection, a water-soluble polymer compound may be added to the blood and the mixture then allowed to stand still. A water-soluble polymer compound in this invention may be a substance which dissolves at a weight/volume % of 0.01 in distilled water or physiological saline at normal temperature of 25 °C. Although the molecular weight of the water-soluble polymer compound is preferably large, a problem may arise whereby, for example, the solution becomes a high viscosity solution and is difficult to mix. Therefore, specifically the average molecular weight is selected from the range of 1,000 to 5,000,000, preferably 3,000 to 1,000,000, more preferably 10,000 to 300,000, and further preferably the range is from 20,000 to 150,000. In this connection, a molecular weight in this invention is determined by a GPC method employing dextran for which the molecular weight is constant as a reference material and distilled water as a solvent.

According to this invention, a water-soluble polymer compound may be a synthetic substance or a naturally occurring substance, or may be a substance obtained by chemically modifying a naturally occurring substance.

According to this invention, a water-soluble polymer compound may be a synthetic substance or a naturally occurring substance, or may be a substance obtained by chemically modifying a naturally occurring substance.

In this invention, a water-soluble polymer compound can be selected from the group consisting of compounds represented by 1) polyamino acids comprising amino acids and/or pharmacologically acceptable salts of amino acids, 2) acidic polysaccharides and/or pharmacologically acceptable salts thereof, and 3) vinyl polymers. To achieve the object of this invention, one kind of the compounds described above can be used or two or more kinds thereof can be combined for use.

As a pharmacologically acceptable salt in this invention, for example, an alkali metal salt such as sodium salt or potassium salt, an alkali earth metal salt such as magnesium salt or calcium salt, a salt formed from inorganic bases such as ammonium salt, or a salt from organic bases such as a diethanolamine salt, cyclohexylamine salt or amino acid salt can be selected for use.

(Polyamino acid) - Examples of a polyamino acid or a pharmacologically acceptable salt thereof used in this invention include a homopolymer or a copolymer in which, for example, α-amino acids, β-amino acids or γ-amino acids such as aspartic acid, glutamic acid, asparagine and histidine are bound by peptide linkage, and there is no particular restriction with respect to D-type, L-type or DL-type. More specifically, polyaspartic acid, polyglutamic acid, polyasparagine, polyhistidine and pharmacologically acceptable salts of these may be exemplified.

Further, according to this invention, in addition to the 20 kinds of amino acids comprising proteins, amino acids and amino acid derivatives such as L-ornithine, a series of α-amino acids, β-alanine, γ-aminobutyric acid, an ω-ester of an acidic amino acid, an N-substitute of a basic amino acid, aspartic acid-L-phenylalanine dimer (aspartame), or aminosulfonic acids such as L-cysteic acid may be included in the main chain or the side chain of the molecule.

Further, these polyamino acids may be substances having a linear structure or substances having a branched structure.

The average molecular weight of a polyamino acid that can be used with this invention is preferably in the range of 1,000 - 5,000,000, and more preferably 1,000 - 1,000,000.

The molecular weight of a polyamino acid can be controlled by adjustment of the polymerization conditions (temperature, time, solvent, catalyst, etc.), and adjustment can also be conducted by recondensing polymers after polymerization using dicyclocarbodiimide (DCC) or the like.

In this invention, it is also possible to use an anhydrous polyacidic amino acid such as, for example, anhydride of polyaspartic acid or anhydride of polyglutamic acid. Of these, from the viewpoint of industrial availability, polysuccinimide, an anhydride of polyaspartic acid, is preferably used.

In the present invention, in particular, acidic polyamino acids can be preferably used. The term "acidic polyamino acid" as used herein refers to a polyamino acid that is negatively charged under a physiological pH condition. A charge state can be grasped by a technique such as isoelectric focusing or titration. Simply, a polyamino acid in which the number of carboxyl groups is greater than the number of amino groups in the ratio of the number of carboxyl groups to amino groups in the molecule can be employed as an acidic polyamino acid.

As the polyamino acid according to this invention, a substance is used in which glutamic acid and/or aspartic acid are comprised at 20% or more, preferably 30% or more, and more preferably 50% or more of the constituent amino acids thereof. In particular, the use of polyaspartic acid or polyglutamic acid is particularly preferred, since the structure thereof is simple and easily synthesized.

The glutamic acid and/or aspartic acid may be randomly distributed in the molecule or may be distributed in a block form. A graft copolymer can also be used.

The polyglutamic acid of this invention may be poly-L-glutamic acid, poly-D-glutamic acid or a mixture of these. An L-type or D-type copolymer can also be used. Similarly, the polyaspartic acid of this invention may be poly-L-aspartic acid, poly-D-aspartic acid or a mixture of these. An L-type or D-type copolymer can also be used.

In this case, when polyaspartic acid is the basic skeleton of the main chain, in some cases the amide bonds in the main chain will be α-bonds and in some cases β-bonds. That is, an α-bond denotes a case where bonding of polyaspartic acid and the copolymer thereof is bonding of aspartic acid or an amino group of copolymer units with a carboxyl group at α-position of aspartic acid, and a β-bond denotes a case where the bonding is with a carboxyl group at β-position of aspartic acid. According to this invention, the form of bonding is not particularly limited, and the bonds may be either α-bonds or β-bonds independently, or may be a mixture of these.

Similarly, when polyglutamic acid is the basic skeleton of the main chain, the amide bonds in the main chain may be α-bonds, γ-bonds or a mixture of both of these kinds of bonds. As with the above, the form of bonding is not particularly limited in this invention.

Of the polyamino acids used in this invention, for example, polyaspartic acid or salts thereof can be prepared according to a method, for example, by hydrolysis of polysuccinimide, by a fermentation method or an enzyme method, or by polymerizing N-carboxy α-amino acid anhydride (NCA) of aspartic acid-4 ester, followed by removing the ester group. Since one part or all of the main chain of polysuccinimide forms normally an imide ring, polyaspartic acid or salts thereof can be obtained by reacting the imide ring with alkali or a nucleophilic reagent to open the ring.

Poly-α-glutamic acid can be obtained, for example, by polymerizing N-carboxylic anhydride of glutamic acid γ-benzyl ester, followed by debenzylating with hydrogen bromide.

A polyamino acid according to this invention may be a substance that is biosynthesized using a microorganism. For example, poly-γ-glutamic acid can also be produced by a fermentation method from microorganisms typically represented by natto bacteria (*bacillus natto*).

(Acidic polysaccharides) - The term "acidic polysaccharides" as used herein refers to those that are negatively charged under a physiological pH condition. A charge state can be easily grasped by a technique such as electrophoresis or titration. As specific examples thereof, a glycosaminoglycan, galacturonic acid and alginic acid may be mentioned. Glycosaminoglycans are defined as a group of acidic polysaccharides that are present broadly in connective tissue to be free or attached to proteins, and contain amino sugars, thus also called mucopolysaccharides. Structurally, glycosaminoglycans have a long chain of disaccharide repeating units comprising amino sugars and uronic acid (or lactose), and comprise both sulfated and nonsulfated substances" (Seikagaku Jiten, 2nd edn., Tokyo Kagaku Doujin (in Japanese)). Typical examples of glycosaminoglycans include hyaluronic acid, chondroitin, chondroitin sulfate, dermatan sulfate, heparin, heparan sulfate and keratan sulfate. In this invention, a substance without a strong action other than an action to separate blood platelets when added into blood is preferable, and since hyaluronic acid fulfills this condition the use thereof is particularly preferable.

Examples of other acidic polysaccharides that are preferably used include dextran derivatives such as dextran sulfate and carboxylated dextran, cellulose derivatives such as carboxymethylcellulose, chitosan derivatives such as carboxymethylchitosan, and guar polycarboxylic acid.

Preparations that are commercially available for use in food or cosmetics or the like (for example, the food grade chondroitin sulfate "mucotein-DK" or the like) can also be utilized. Further, in this invention, pharmacologically acceptable salts of acidic polysaccharides are also preferably used.

(Water-soluble vinyl polymers) - Examples of water-soluble vinyl polymers that can be used in this invention include polyacrylic salts such as polyvinyl pyrrolidone, polyacrylic acid, sodium polyacrylate and ammonium polyacrylate, polymethacrylic acid and salts thereof, polystyrene sulfonate and salts thereof, polyvinyl sulfonate, polyvinyl acetate, polyvinyl alcohol, polyacrylamide, polyvinylamine, polyallylamine, polyallyl alcohol, polyvinyl alcohol, ethyleneimine-based polymer acid salt, polyamidine and polyisoprene sulfonate, as well as their respective derivatives. Further examples thereof may be mentioned, and the water-soluble vinyl polymers are not particularly limited.

Among the water-soluble vinyl polymers, an acid vinyl polymer and a pharmacologically acceptable salt thereof are more preferably used. The term "acidic vinyl polymer" as used herein refers to a vinyl polymer that is negatively charged under a physiological pH condition, and more specifically a vinyl polymer that includes an acidic group such as carboxyl group, sulfonate group, or phosphate group in the molecule is preferable, for example, polyacrylic acid, polyacrylic acid, sodium polyacrylate, ammonium polyacrylate, polymethacrylic acid and salts thereof, polystyrene sulfonate and salts thereof, polyvinyl sulfonate or polyisoprene sulfonate.

(Method of preparing platelet-rich plasma) - To prepare the platelet-rich plasma of this invention, to a blood volume of 1.5 ml that includes an anticoagulant, 0.0015 - 150 mg of water-soluble polymer compound, preferably 0.15 - 45 mg, and more preferably 1.5 - 4.5 mg can be added. When converted, this is equivalent to approximately 0.0001 - 10% w/v, preferably 0.01 - 3% w/v, and more preferably 0. 1 - 0.3% w/v. When preparing a larger volume of platelet-rich plasma, the blood volume can be increased and the water-soluble polymer compound can be added in the same proportion as above.

The water-soluble polymer compound can be added to a container into which collected blood is to be added, or the compound can be added directly into a syringe used for blood collection.

By adding a water-soluble polymer compound of an amount selected from the aforementioned range to blood including an anticoagulant and gently mixing so that the compound diffuses throughout the blood, and allowing the blood to stand, sedimentation of red blood cells in a selective and accelerative manner can be achieved, and platelet-rich plasma that includes not only blood platelets, but also blood components such as blood plasma components and white blood cells can be obtained in the supernatant. By achieving sedimentation of red blood cells at the latest within 3 hours, preferably within 2 hours, and at the earliest about 10 minutes after the start of treatment, it is possible to obtain the platelet-rich plasma that is the object of this invention in approximately 20 to 30 minutes.

Further, when separation of red blood cells is insufficient or when it is necessary to conduct separation in a shorter time, it is also possible to conduct a step of weak centrifugation that is of a degree that does not cause the conventional coagulation of red blood cells. More specifically, a step of centrifugation at 142 G for 5 minutes or less can be added.

(Use of platelet-rich plasma by this invention) - Platelet-rich plasma prepared according to the method of this invention can be applied as a healing accelerator for all kinds of tissue and organs, such as a wound-healing agent, additive for bone augmentation in the periphery of a dental implant, additive for use when transplanting bone or artificial bone to a bone defect site, wound healing accelerator, therapeutic agent for dermatosis, therapeutic agent for cutaneous ulcers, an accelerator of tissue repair after therapy or treatment for plastic and/or cosmetic purposes, postoperative tissue repair agent, postoperative tissue repair agent for an orthopedic region, and agent for nerve tissue repair. That is, the aforementioned disorders or damage to skin or tissue can be treated by the platelet-rich plasma by this invention.

The platelet-rich plasma by this invention can be applied not only for humans, but also for non-human mammals. Examples of the mammals include animals living above ground in particular, and the platelet-rich plasma can be applied to dogs, cats, hamsters and the like that are generally kept as pets. The platelet-rich plasma can also be applied to animals that are employed for sports such as racehorses and fighting bulls.

The platelet-rich plasma prepared from a patient's own blood according to the method of this invention can be used for the objects of the aforementioned therapy or treatment. It is also of course possible to use platelet-rich plasma that is not derived from a patient's own blood as long as the blood group corresponds.

As a specific method of use, a required amount of the platelet-rich plasma by this invention can be administered to a treatment site by a method such as coating or injecting.

(Reagent, instrument and kit for preparation) - The main feature of this invention is that, as described in the foregoing, it is possible to conveniently and easily prepare platelet-rich plasma that is effective for therapy and the like from a patient's own blood. Convenient and easy preparation is achieved by providing a water-soluble polymer compound that is used in the preparation method of this invention as a reagent.

More specifically, reagents acquired by adding the various kinds of water-soluble polymer compounds described in the above "Water-soluble polymer compound" section into suitable containers such as vials or ampoules, and a reagent kit comprising substances or solutions or the like containing a plurality of these reagents are also included in this invention. Further, specific examples of instruments used in the preparation method of this invention comprise a blood collection tube or syringe used for collection of blood, and a sterilized test tube or container made of plastic or the like for adding a water-soluble polymer compound to collected blood. Examples of the kit for preparing platelet-rich plasma by this invention include a kit composed of reagents and instruments selected from the reagents and instruments exemplified in the foregoing. The use of this kit enables easy preparation of the platelet-rich plasma of this invention at the bedside where collection of blood is performed or the like.

Further, an instrument in which a water-soluble polymer of this invention is added in advance into a syringe or container used when collecting blood is also preferably used. An instrument that is particularly preferably used is one in which the water-soluble polymer has been previously dissolved in sodium citrate or ACD that are widely used as anticoagulants, after which the solution has been added into the aforementioned syringe or container.

### Examples

This invention is described in further detail hereunder by way of examples and experimental examples, although the invention is not limited to these examples.

### Example 1

(Object) - The object of this example was to examine the appearance of supernatant and effect on sedimentation of red blood cells produced by addition to whole blood (peripheral blood) of sodium poly-L-glutamate (molecular weight 21,270) (manufactured by Sigma Chemical Co.), and measure the number of cells in the supernatant and observe the shape of cells using a differential interference microscope.

(Materials and Method) - Four kinds of samples were prepared by the following method to compare differences in the rate of red blood cell sedimentation. An anticoagulant was prepared to contain sodium citrate at 3.13 w/v%.
Sample 1: 2 mg of sodium poly-L-glutamate was added to 1.5 ml of blood.
Sample 2: 1.35 ml of blood was added to 0.15 ml of the sodium citrate solution to form a total volume of 1.5 ml, and 2 mg of sodium poly-L-glutamate was then added thereto.
Sample 3: 1.35 ml of blood was added to 0.15 ml of the sodium citrate solution to form a total volume of 1.5 ml.
Sample 4: 2 mg of sodium poly-L-glutamate was added to 1.5 ml of phosphate buffer (PBS).

### (Results)

1) Sedimentation of red blood cells
The state of sedimentation of red blood cells after allowing each of the above samples to stand for 30 minutes was as follows.
For sample 1, blood coagulation occurred during the observation, and sedimentation of red blood cells could not be achieved. In comparison to sample 3, because sample 2 contained sodium poly-L-glutamate, acceleration of sedimentation of red blood cells was achieved, and supernatant was observed to appear.
2) Platelet count
The number of platelets in the supernatant after allowing each of the above samples to stand for 30 minutes was measured by the fully automatic hematology analyzer Celltac alpha (MEC-6318) manufactured by Nihon Kohden Corporation. The platelet count (10⁴/µl) in the supernatant of each sample is shown in Table 1.

**(Table 1)**

| | Whole blood (peripheral blood) | Sample 1 | Sample 2 | Sample 3 |
|---|---|---|---|---|
| Platelet count | 19.5 | 1 | 31.7 | 12.8 |

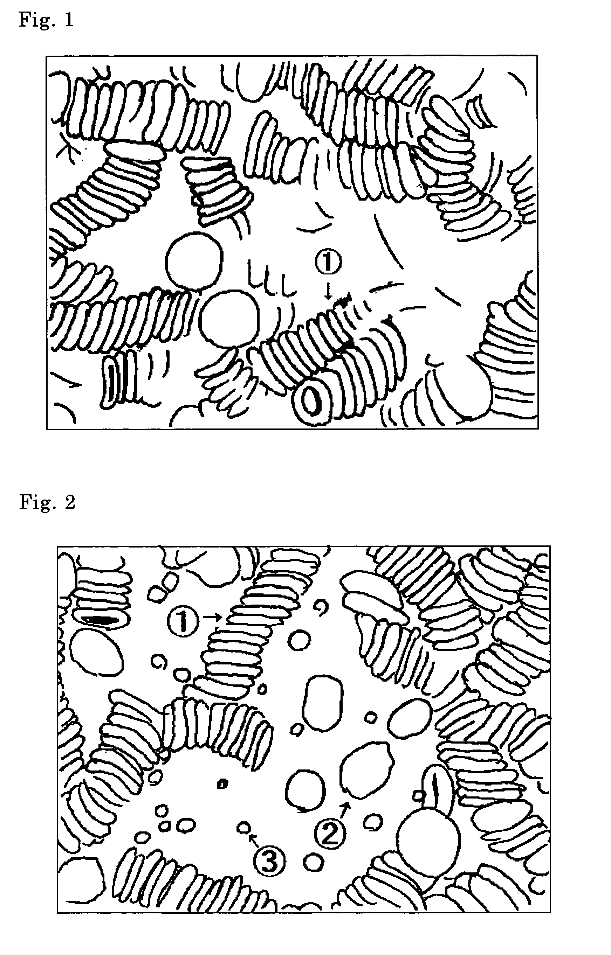

3) Microscopic observation (Figures 1 to 5)
The supernatant portion of each sample was observed using incident-light fluorescence and a differential interference microscope (Nikon Eclipse E600), and photographed.

Figure 1 and Figure 2 are views that schematically show photographs obtained with a differential interference microscope of peripheral blood and the supernatant portion of sample 3, respectively, at a magnification of 1000 times. Red blood cells, white blood cells and blood platelets could be classified comparatively easily based on size, color and shape by this microscopic observation.

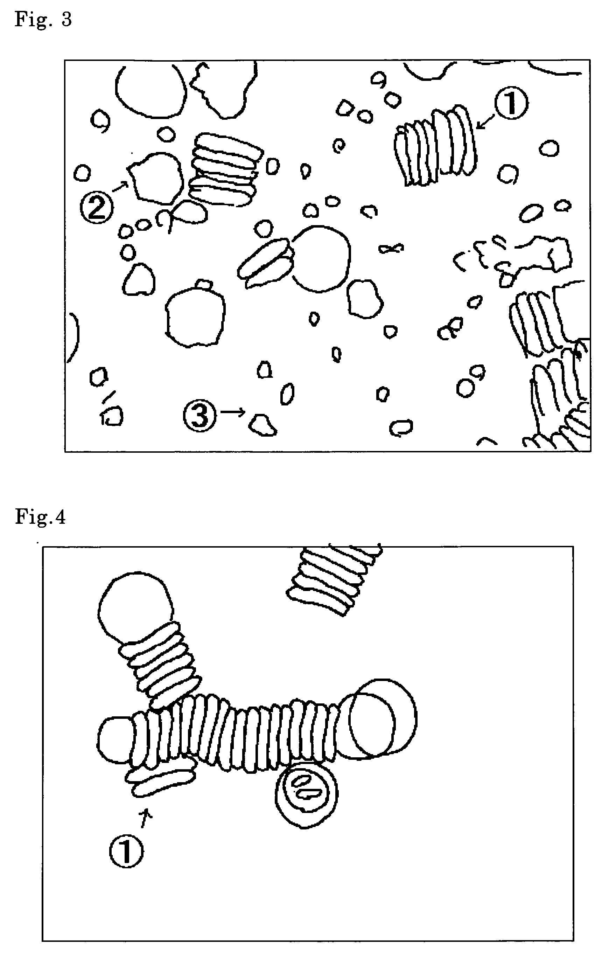

Figure 3 is a schematic diagram of the image of the supernatant obtained in sample 2. In comparison to Figure 1, there were fewer red blood cells and more blood platelets and white blood cells were observed.

Figure 4 is a schematic diagram of the image of the supernatant of sample 1 in which blood coagulation was observed and blood platelets were not observed at all.

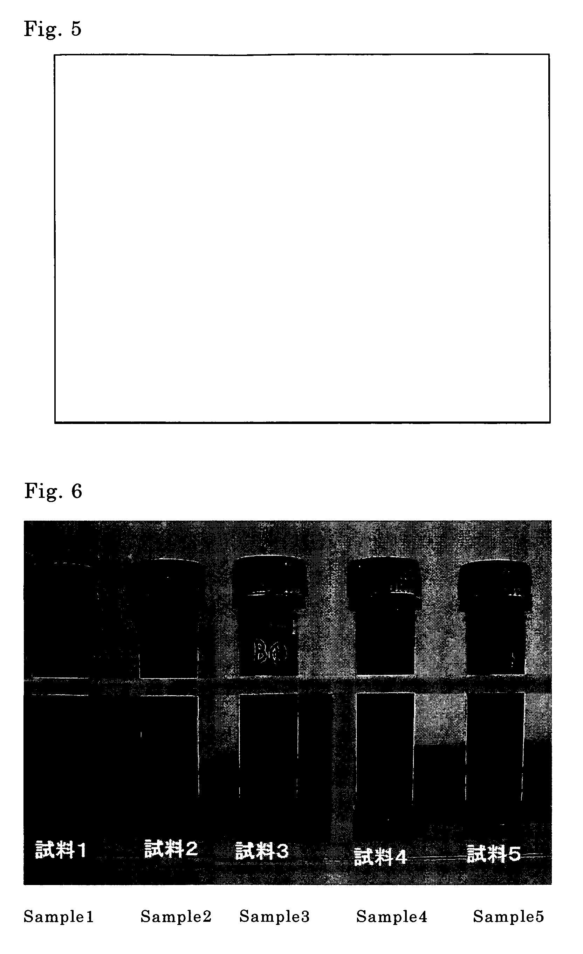

Figure 5 is a schematic diagram of the image of sample 4, in which nothing could be observed.

These results suggested that addition of sodium poly-L-glutamate to whole blood containing an anticoagulant led to the existence of blood cell components containing many blood platelets in supernatant of the red blood cell sediment.

### Example 2

(Object) - The object of this example was to examine the effect achieved by addition of sodium poly-L-glutamate (molecular weight 21,270) (manufactured by Sigma Chemical Co.) in cases where the molecular weight was the same but the added amount was changed.

(Materials and Method) - Five kinds of samples were prepared by the following method. 1.35 ml of blood was added to 0.15 ml of an aqueous solution containing sodium citrate at 3.13 w/v% as an anticoagulant, to obtain a total volume of 1.5 ml for use as whole blood samples. Various amounts of sodium poly-L-glutamate (Sigma Chemical Co.) were added to the whole blood samples.

| | |
|---|---|
| Sample 1: sodium poly-L-glutamate | 1mg |
| Sample 2: sodium poly-L-glutamate | 2mg |
| Sample 3: sodium poly-L-glutamate | 2.5mg |
| Sample 4: sodium poly-L-glutamate | 3mg |
| Sample 5: sodium poly-L-glutamate | 4mg |

### (Results)

1) Sedimentation of red blood cells
   After each of the above samples was allowed to stand for 30 minutes, the largest effect on the sedimentation of red blood cells was observed in the sample to which 3 mg of sodium poly-L-glutamate was added. The amount of supernatant for that sample was 0.8 ml.
2) Platelet count
   The number of platelets in the supernatant of each of the above samples after the samples were allowed to stand for 30 minutes was measured in the same manner as Example 1. While the platelet count for the whole blood sample (peripheral blood) was 19.7 (10⁴/µl), the platelet count in the supernatant of each sample was from 34.5 (10⁴/µl) to 37.0 (10⁴/µl), representing an almost twofold increase from the whole blood sample.

### Example 3

(Object) - The object of this example was to examine the effect obtained when sodium poly-L-glutamate of different molecular weights were added to blood.

(Materials and Method) - 1.35 ml of blood was added to 0.15 ml of an aqueous solution containing sodium citrate at 3.8 w/v% as an anticoagulant, to obtain a total volume of 1.5 ml for use as whole blood samples. 3 mg of sodium poly-L-glutamate (Sigma Chemical Co.) of different molecular weights were added thereto to prepare 5 kinds of samples. The rates of sedimentation of red blood cells when each of the above samples was allowed to stand for 30 minutes were compared.
Sample 1: sodium poly-L-glutamate not added (control)
Sample 2: sodium poly-L-glutamate (molecular weight 5,800)
Sample 3: sodium poly-L-glutamate (molecular weight 17,500)
Sample 4: sodium poly-L-glutamate (molecular weight 21,270)
Sample 5: sodium poly-L-glutamate (molecular weight 42,000)

### (Results)

1) Sedimentation of red blood cells
The results of observing the state of sedimentation of red blood cells showed that the rate of sedimentation was faster in the samples in which the molecular weight of sodium poly-L-glutamate was larger, and a greater supernatant amount was also confirmed for those samples.
2) Measurement of blood cells
When the concentrations of platelets in the supernatants of sample 4 and sample 5 were compared, it was found that the concentration was higher for sample 5 which had the larger molecular weight.

**(Table 2)**

| | Blood platelets (10⁴/µl) | White blood cells (10²/µl) | Red blood cells (10⁴/µl) | Volume of entire blood occupied by supernatant (%) |
|---|---|---|---|---|
| Control (blood)* | 20.7 | 69 | 424 | |
| Control (supernatant)** | 45.1 | 118 | 26 | 11.2 |
| Sample 2 | 44.6 | 141 | 6 | 11.4 |
| Sample 3 | 47.5 | 147 | 3 | 14.3 |
| Sample 4 | 37.0 | 90 | 3 | 48.9 |
| Sample 5 | 38.6 | 77 | 2 | 50 |

| | | | | |
|---|---|---|---|---|
| * Peripheral blood | | | | |
| * Supernatant after red blood cell sedimentation of peripheral blood | | | | |

### Example 4

(Object) - The object of this example was to examine the effect obtained when various kinds of polyamino acids were added to blood.

(Materials and Method) - Five kinds of samples were prepared by the following method.

1.35 ml of blood obtained by blood collection was added to 0.15 ml of an aqueous solution containing sodium citrate at 3.8 w/v% as an anticoagulant, to obtain a total volume of 1.5 ml for use as whole blood samples.

Five kinds of whole blood samples were prepared respectively for blood obtained from a test subject A and blood from a test subject B, and the following polyamino acids were added at each weight to the whole blood samples.
Sample 1: no polyamino acid added (control)
Sample 2: poly-L-histidine (molecular weight 20,000) (Sigma Chemical Co.) 3mg
Sample 3: sodium poly-L-aspartate (molecular weight 8,300) (Sigma Chemical Co.) 3mg
Sample 4: sodium poly-L-aspartate (molecular weight 35,700) (Sigma Chemical Co.) 3mg
Sample 5: sodium poly-L-glutamate (molecular weight 53,785) (Sigma Chemical Co.) 3mg

The appearance of supernatant and platelet count was compared for each of the above samples after the samples were allowed to stand for 30 minutes.

### (Results)

1) Supernatant
Supernatant was observed in each of samples 2 to 4 after the samples were allowed to stand for 30 minutes. The largest amount of supernatant was observed in sample 5, followed by samples 3, 4 and 2 in that order. Figure 6 shows the results when each of the samples for test subject A was allowed to stand for 1 hour.
2) Platelet count
Table 3 shows the results obtained when the number of platelets in the above supernatants was measured in the same manner as Example 1. It was found that a large amount of platelets were observed in the supernatant of each of samples 2 to 4.

**(Table 3)**

| | Sample 1 (peripheral blood) | Sample 2 | Sample 3 | Sample 4 | Sample 5 |
|---|---|---|---|---|---|
| Test subject A | 18.7 | 44.1 | 38.4 | 43.5 | 37.5 |
| Test subject B | 24.3 | 44.3 | 39.2 | 45.8 | 37.1 |
| Platelet count (10⁴/µl) | | | | | |

### Example 5

(Object) - The object of this example was to examine the effect obtained when sodium hyaluronate that is one kind of acidic polysaccharide was added to blood.

(Materials and Method) - 1.35 ml of blood was added to 0.15 ml of an aqueous solution containing ACD at 3.8 w/v% as an anticoagulant, to obtain a total volume of 1.5 ml for use as whole blood samples. Various amounts of sodium hyaluronate (derived from cockscomb; manufactured by Wako Pure Chemical Industries, Ltd.) were added thereto to prepare 5 kinds of samples.
Sample 1: sodium hyaluronate not added (control)
Sample 2: sodium hyaluronate 1mg
Sample 3: sodium hyaluronate 2 mg
Sample 4: sodium hyaluronate 3 mg
Sample 5: sodium hyaluronate 4 mg

The appearance of supernatant and platelet count was compared for each of the above samples after the samples were allowed to stand for 40 minutes.

### (Results)

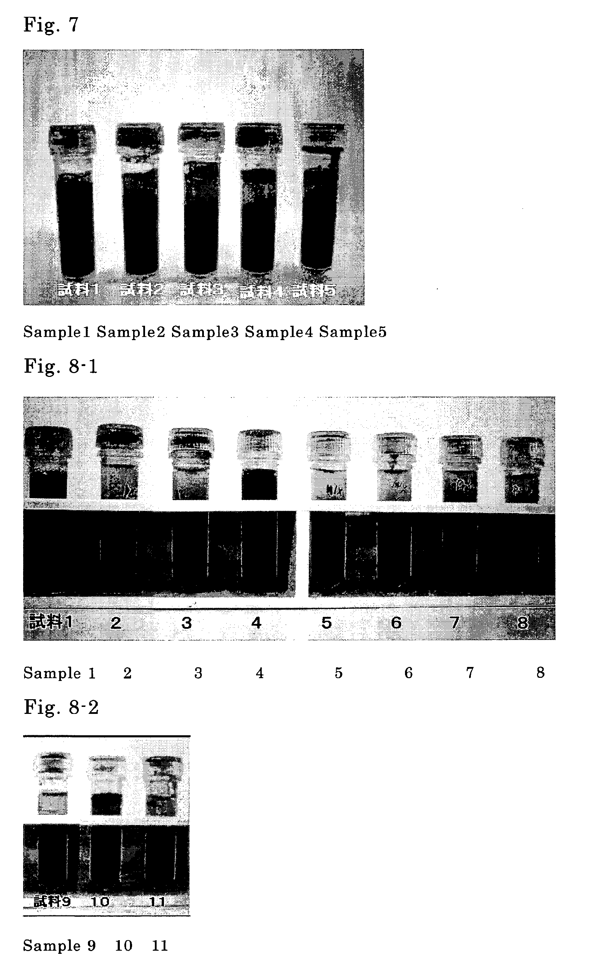

1) Supernatant
Observation of the supernatant amount after the samples were allowed to stand for 40 minutes showed that although the supernatant amount increased as the amount of sodium hyaluronate added increased, it was less in comparison to that for sodium poly-L-glutamate. Figure 7 shows the results after the samples were allowed to stand for 40 minutes.
2) Platelet count
Table 4 shows the results obtained when the number of platelets in the supernatants obtained after each of the above samples was allowed to stand for 40 minutes was measured in the same manner as Example 1.

**(Table 4)**

| | | Sample 1 (peripheral blood) | Sample 2 | Sample 3 | Sample 4 | Sample 5 |
|---|---|---|---|---|---|---|
| | Platelet count (10⁴/µl) | 18.7 | 23.6 | 18.5 | 24.3 | 20.8 |

### Example 6

(Object) - The object of this example was to investigate the effect obtained when sodium poly-L-glutamate and polyacrylic acid were added to blood.

(Materials and Method) - 1.35 ml of blood was added to 0.15 ml of an aqueous solution containing ACD at 3.8 w/v% as an anticoagulant, to obtain a total volume of 1.5 ml for use as whole blood samples. Four kinds of samples were prepared by adding thereto polyacrylic acid or sodium poly-L-glutamate.
Sample 1: water-soluble polymer compound not added (control)
Sample 2: polyacrylic acid (molecular weight 2,000) (manufactured by Aldrich Chem. Co.) 3 mg
Sample 3: polyacrylic acid (molecular weight 2,000) (Aldrich Chem. Co.) 4 mg
Sample 4: sodium poly-L-glutamate (molecular weight 53,785) (Sigma Chemical Co.) 3 mg

Each of the above samples was allowed to stand to observe the appearance of supernatant after 30 minutes and 50 minutes, and the number of platelets in each supernatant after 2 hours was measured.

### (Results)

1) Supernatant
After 30 minutes, although a difference with the control was not observed in samples 2 and 3, a large amount of supernatant was observed to appear in sample 4 in comparison with the control.
After 50 minutes, the amount of supernatant was large compared to the control even in samples 2 and 3 to which the polyacrylic acid had been added.
2) Platelet count
Table 5 shows the results obtained when the number of platelets in each of the above supernatants was measured in the same manner as Example 1. While the platelet count was large for each of samples 2 to 4 compared to sample 1, a large platelet count was observed for samples 2 and 3 in particular.

**(Table 5)**

| | Sample 1 (peripheral blood) | Sample 2 | Sample 3 | Sample 4 |
|---|---|---|---|---|
| Platelet count (10⁴/µl) | 19.6 | 51.1 | 52.5 | 32.2 |
| Volume of supernatant/entire blood after 5 hours (%) | | 30.8 | 30.8 | 50 |

### Example 7

(Object) - The object of this example was to examine the effect obtained when various kinds of water-soluble polymer compounds were added to blood.

(Materials and Method) - 1.35 ml of blood was added to 0.15 ml of an aqueous solution containing ACD at 3.8 w/v% as an anticoagulant, to obtain a total volume of 1.5 ml for use as whole blood samples. Eleven kinds of samples were prepared by adding various kinds of polymer compounds of various weights to respective whole blood samples.
Sample 1: Water-soluble polymer compound not added (control)
Sample 2: sodium poly-L-glutamate (molecular weight 53,785) (Sigma Chemical Co.) 3 mg
Sample 3: sodium polyaspartate (molecular weight 35,700) (Sigma Chemical Co.) 3 mg
Sample 4: polyasparagine (molecular weight 10,700) (Sigma Chemical Co.) 3 mg
Sample 5: mixture of sodium poly-L-glutamate (molecular weight 53,785) (Sigma Chemical Co.) 1.5 mg and sodium polyaspartate (molecular weight 35,700) (Sigma Chemical Co.) 2.5mg
Sample 6: mixture of sodium poly-L-glutamate (molecular weight 53,785) (Sigma Chemical Co.) 2.25 mg and sodium polyaspartate (molecular weight 35,700) 0.75 mg
Sample 7: poly(sodium glutamate-tyrosine) (4:1)) (Sigma Chemical Co.) 2 mg
Sample 8: poly(sodium glutamate-tyrosine) (4:1)) (Sigma Chemical Co.) 4 mg
Sample 9: sodium carboxymethyl cellulose (CMC50) (Gotoku Chemical Company, Ltd.) 2 mg
Sample 10: mixture of sodium hyaluronate (Wako Pure Chemical Industries, Ltd.) 1.5 mg and sodium poly-L-glutamate (molecular weight 53,785) (Sigma Chemical Co.) 1.5 mg
Sample 11: mixture of sodium hyaluronate (Wako Pure Chemical Industries, Ltd.) 1.8 mg and sodium poly-L-glutamate (molecular weight 53,785) (Sigma Chemical Co.) 1.2 mg

The appearance of supernatant and platelet count after each of the above samples was allowed to stand for 30 minutes were compared.

### (Results)

1) Supernatant
   Observation of the supernatant obtained after each of the above samples was allowed to stand for 30 minutes showed that although almost no supernatant was observed to appear in sample 4 to which polyasparagine was added, the amount of supernatant in the other sample was at the same level as that of sodium polyglutamate or slightly less than for sodium polyglutamate. After being allowed to stand for 2 hours, supernatant was also observed to appear in sample 4.
   The results after the samples were allowed to stand for 30 minutes are shown in Figures 8-1 and 8-2.
2) Platelet count
   Table 6 shows the results obtained when the number of platelets in supernatants obtained after each of the above samples was allowed to stand for 30 minutes was measured in the same manner as Example 1.

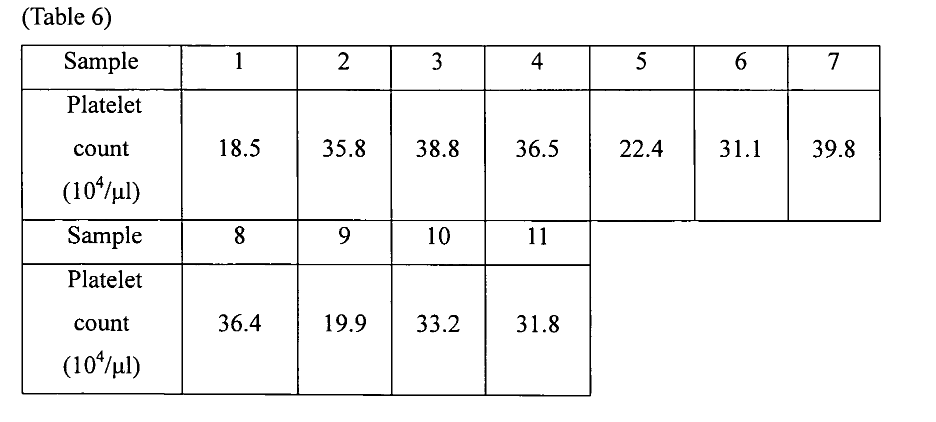

### Example 8

(Object) - The object of this example was to examine the effect obtained when various kinds of water-soluble polymer compounds were added to blood.

(Materials and Method) - Eight kinds of samples were prepared by the following method. 1.35 ml of blood was added to 0.15 ml of an aqueous solution containing ACD at 3.8 w/v% as an anticoagulant, to obtain a total volume of 1.5 ml for use as whole blood samples. The following polymer compounds of various weights were added to respective whole blood samples.
Sample 1: water-soluble polymer compound not added (control)
Sample 2: sodium poly-L-glutamate (molecular weight 53,785) (Sigma Chemical Co.) 3 mg
Sample 3: sodium polyaspartate (molecular weight 35,700) (Sigma Chemical Co.) 3 mg
Sample 4: sodium hyaluronate (Wako Pure Chemical Industries, Ltd.) 3 mg
Sample 5: dextran sulfate (MDS Kowa) (Kowa Co., Ltd.) 6mg
Sample 6: sodium polygalacturonate (Sigma Chemical Co.) 3 mg
Sample 7: sodium poly γ-D,L-glutamate (straight chain type) (Meiji Seika Kaisha, Ltd.) 3 mg
Sample 8: sodium poly γ-D,L-glutamate salt (crosslinking type) (Meiji Seika Kaisha, Ltd.) 3 mg

The appearance of supernatant and platelet count after each of the above samples was allowed to stand for 30 minutes were compared.

### (Results)

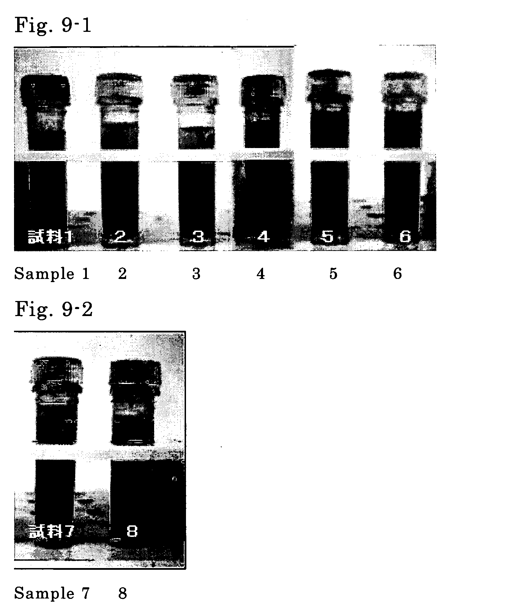

1) Supernatant
   After each of the above samples was allowed to stand for 30 minutes, supernatant was observed to appear in all of the samples except samples 5 and 6. Figures 9-1, 9-2 and 9-3 show the results after the samples were allowed to stand for 30 minutes. After 2 hours, supernatant was also observed to appear in samples 5 and 6.
2) Platelet count
   Table 7 shows the results obtained when the number of platelets in supernatants obtained after each of the above samples was allowed to stand for 30 minutes (about 2 hours after treatment for samples 5 and 6) was measured in the same manner as Example 1.

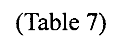

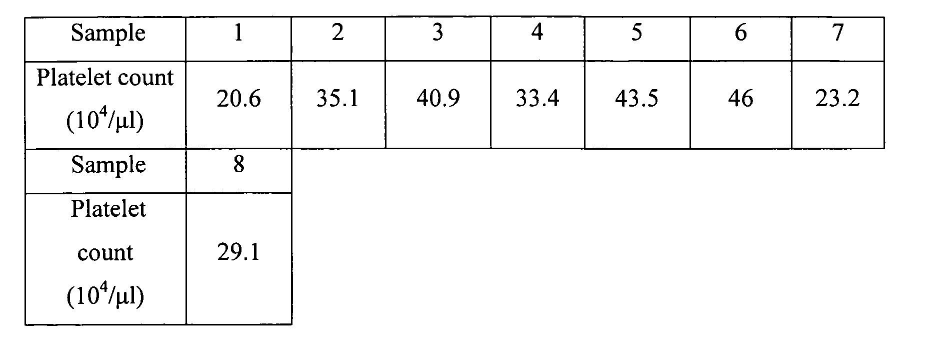

### Experimental Example 1

(Object) - The object of this experimental example was to examine the function of platelets in platelet-rich plasma obtained by the method of this invention. Since platelets that have a high level of function coagulate and sediment together with fibrin clots, A sample (of supernatant) containing such kind of platelets is high in transmittance. The measurement of platelet function can be conducted utilizing this principle.

(Materials and Method) - A substance obtained by adding 10 µL (12.21 g/L) of calcium chloride and 40 µL (0.2 mmol) of ADP to 390 µL of platelet-rich plasma obtained according to the method of this invention was employed as a measurement sample. The transmittance of supernatant collected according to the method of this invention was determined with a spectrophotometer (MCM Hema Tracer 212 (MCMEDICAL)) at a wavelength of 690 nm. The term "platelet-rich plasma obtained according to the method of this invention" as used in this example refers to supernatant obtained by adding sodium poly-L-glutamate (3 mg) to the above starting material and allowing the mixture to stand for 30 minutes.

### (Results)

Since the supernatant collected according to the method of this invention contained a large amount of white blood cells and blood platelets in a considerably suspended state, this supernatant was centrifuged for 10 minutes at 142 G (1200 rpm) to sediment blood cell components, and the top layer part was then employed as a reference transmittance (100%).

The transmittance of a supernatant part obtained after adding calcium chloride and ADP and allowing the sample to stand for 30 minutes reached 100 %, thus clarifying that blood platelets obtained by the method of this invention retained a high agglutination function.

### Experimental Example 2

(Object) - The object of this experimental example was to measure the amount of fibrinogen in supernatant (platelet-rich plasma) obtained by the method of this invention and by the conventional method. The presence of fibrinogen, the precursor of fibrin that serves as a scaffold at the time of cell migration is extremely important. The amount of fibrinogen in platelet-rich plasma prepared by the method of this invention and by the conventional method was measured.

(Materials and Method) - A substance having a total volume of 1.5 ml obtained by adding 1.35 ml of whole blood to 0.15 ml of a solution containing sodium citrate at 3.8 w/v% as an anticoagulant was used as starting material. The term "platelet-rich plasma obtained by the method of this invention" as used in this example refers to supernatant obtained by adding sodium poly-L-glutamate salt (3 mg) to the aforementioned starting material and allowing the mixture to stand for 30 minutes.
Sample 1: supernatant collected according to the method of this invention
Sample 2: top layer of plasma obtained after centrifuging starting material 1 time (platelet poor plasma: PPP) (centrifugation condition: 1000 G × 10 min)
Sample 3: plasma obtained after centrifuging starting material 2 times (platelet-rich plasma: PRP) (centrifugation conditions: 1000 G × 10 min and 700 G × 8 min) 0.5 ml of each sample was collected to determine the fibrinogen amount by a measurement method that used a light scattering method.

(Results) - The fibrinogen amounts measured were 222 mg/dl in sample 1, 252 mg/dl in the PPP of sample 2 and 178 mg/dl in the PRP of sample 3. It was thus clarified that the fibrinogen amount in supernatant (platelet-rich plasma) obtained by the method of this invention was greater than that in PRP produced by the conventional method.

### Experimental Example 3

(Object) - The object of this experimental example was to determine the count of each kind of blood cell and the platelet recovery rate for platelet-rich plasma prepared according to the conventional method (hereunder, referred to as "conventional PRP") and platelet-rich plasma prepared according to the method of this invention (hereunder, referred to as "PRP of the method of this invention").

(Materials and Method) - The conventional PRP was prepared using a PRP kit (manufactured by Curasan, Pharma GmbH) employing as starting material a substance having a total volume of 8.5 ml obtained by adding blood to 0.85 ml of a solution containing ACD at 3.8 w/v% as an anticoagulant. A Heraeus Labofuge 300 was used as the centrifuge. Supernatant was fractionated by conducting a first centrifugation step at 3600 rpm for 15 minutes and a second centrifugation step at 2400 rpm for 10 minutes, whereby approximately 0.7 ml of platelet-rich plasma was collected.

For the PRP of the method of this invention, a substance having a total volume of 1.5 ml obtained by adding 1.35 ml of blood to 0.15 ml of solution containing ACD at 3.8 w/v% as an anticoagulant was used as starting material. 3 mg of sodium poly-L-glutamate salt (molecular weight 53,785) was added to the starting material, which was allowed to stand for 30 minutes to fractionate supernatant, whereby approximately 0.75 ml of platelet-rich plasma was collected.

(Results) - Table 8 shows the result of each measurement. The platelet recovery rate was determined according to the formula below. As a result, it was confirmed that the platelet-rich plasma obtained by the method of this invention was obtained more efficiently and using a smaller amount of blood in comparison to the platelet-rich plasma obtained by the conventional method.

Method for calculating recovery rate = (count of recovered platelets/platelet count at time of blood collection) × (collected PRP amount/collected blood amount)

**(Table 8)**

| | Control | Conventional PRP | PRP of method of this invention |
|---|---|---|---|
| White blood cells (10²/µl) | 52 | 53.5 | 80 |
| Red blood cells | 351 | 8 | 2 |
| (10⁴/µl) | | | |
| Platelets (10⁴/µl) | 18 | 67.2 | 33 |
| Platelet recovery rate | | 30.7% | 91.7% |

### Experimental Example 4

(Object) - The object of this experimental example was to compare healing effects for nude mice treated with PRP of the method of this invention (group of this invention) and a group that was not administered with any substance (control group).

### (Materials and Method)

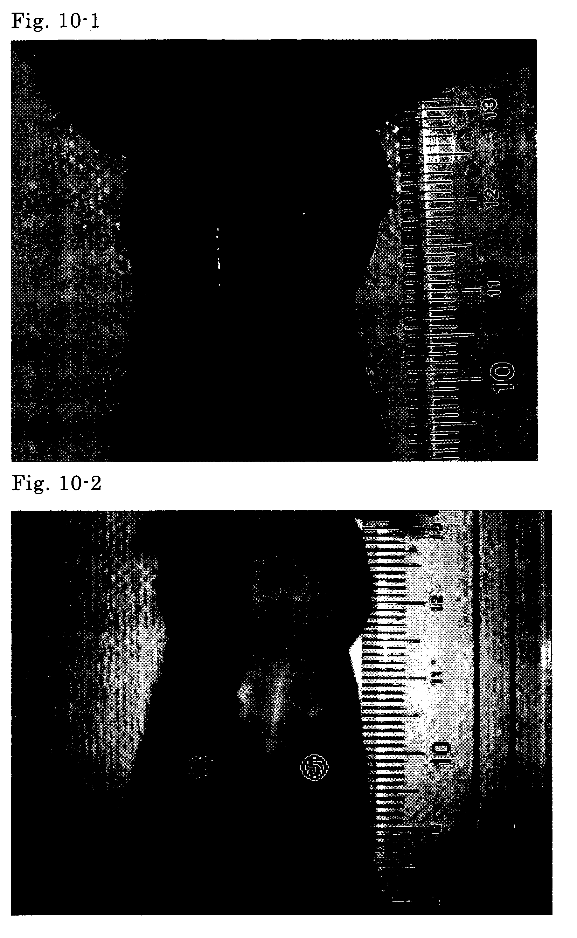

The PRP of the method of this invention was prepared according to the method of Experimental Example 3. The following treatment was carried out using a total of 10 immunodeficient mice BALB/c-nu/nu (6-11 weeks).
1) 10mm incisions were made at 2 locations on the back of each mouse, and epithelium was detached for 5 mm from there towards the outer side to create an incision wound (Figure 10-1).
2) Into the incision wound on the right side of the photograph was added 50 mg of coagulated platelet-rich plasma obtained by adding 5% calcium chloride (about 0.07 ml) to PRP (about 0.7 ml) of the method of this invention collected from human blood to activate the PRP, after which double armed suture was carried out with nylon thread (4-0).
3) Double armed suture was carried out on the incision wound on the left side of the photograph without the addition of a substance, and this was employed as the control group.

(Results) - From the fourth day to seventh day after the surgery, both the length and width of the wound part of the group of this invention was smaller in comparison to the control group, clearly demonstrating a healing accelerating effect (Figure 10-2).

### Experimental Example 5

(Object) - The object of this experimental example was to compare healing effects for nude mice treated with PRP of the method of this invention and a group administered with conventional PRP. The respective platelet-rich plasmas were prepared according to the method of Experimental Example 3.

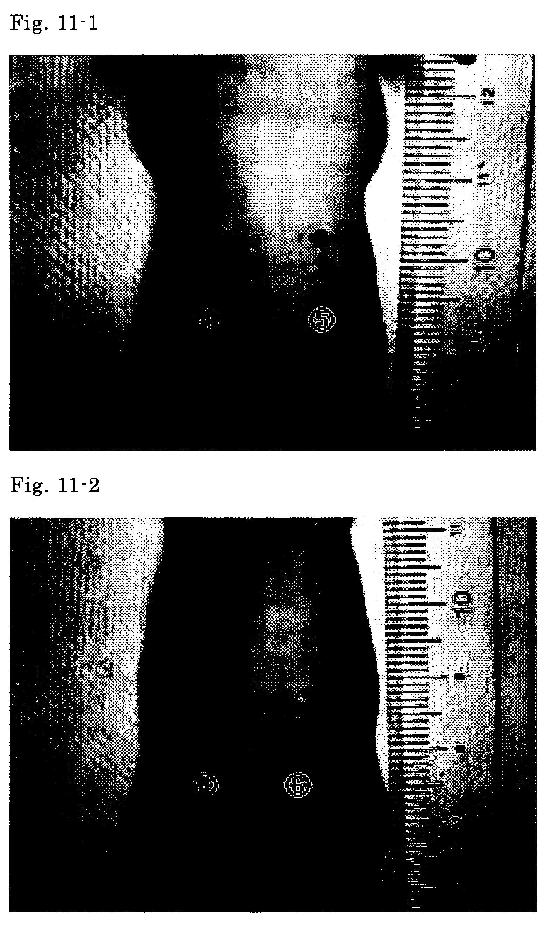

(Materials and Method) - A total of 2 immunodeficient mice BALB/c-nu/nu (11 weeks) were used.
1) 10mm incisions were made at 4 locations on the back of each mouse, and epithelium was detached for 5 mm from there towards the outer side to create an incision wound.
2) Into the incision wound on the upper right side of the photograph of 2 mice was added 50 mg of coagulated platelet-rich plasma obtained by activating PRP of the method of this invention collected from human blood in the same manner as Experimental Example 4, after which double armed suture was carried out with nylon thread (4-0) (Figure 11-1). For the incision wound on the lower right side of the photograph, conventional PRP was activated and an equal amount thereof was added to the wound part (Figure 11-2).
3) Nothing was added to the incision wounds on the upper and lower areas on the left side of the photograph, and the wounds were sutured and employed as a control side.

(Results) - Both the length and width of the incision wound to which the PRP of the method of this invention was added were smaller than that added with the conventional PRP, indicating a high healing accelerating effect for the PRP of the method of this invention in comparison to the conventional PRP (Figures 11-1 and 11-2).

### Experimental Example 6

(Object) - The object of this experimental example was to compare the healing accelerating effect of conventional PRP with a substance obtained by adding white blood cells to the conventional PRP. The conventional PRP was prepared according to method of Experimental Example 3.

The PRP of the method of this invention contains not only an increased amount of platelets, but also an increased amount of white blood cells, and it is considered that this further enhances the healing accelerating effect. Therefore, white blood cells were also added to the conventional PRP to confirm how the added white blood cells affected healing.

(Materials and Method) - A total of 4 immunodeficient mice BALB/c-nu/nu (11 weeks) were used.
1) 10mm incisions were made at 4 locations on the back of each mouse, and epithelium was detached for 5 mm from there towards the outer side to create an incision wound.
2) Into the incision wound on the upper right side of the photograph of 2 mice was added 50 mg of coagulated platelet-rich plasma obtained by activating conventional PRP prepared from human blood in the same manner as Experimental Example 5, after which double armed suture was carried out with nylon thread (4-0) (Figure 12-1). For the other 2 mice, white blood cells were mixed with conventional PRP obtained in the same manner, the conventional PRP + white blood cells (WBC) was activated, and an equal amount thereof was added to the wound part (Figure 12-2).
3) Nothing was added to the incision wounds on the upper and lower areas on the left side of the photograph, and the wounds were sutured and employed as a control side.

Conventional PRP was added to the incision wound on the right side of the photograph of the mouse shown in Figure 12-1. The composition of the conventional PRP was a white blood cell count of 53 × 10²/µl and a platelet count of 67.1 × 10⁴/µl.

Conventional PRP + white blood cells (WBC) was added to the incision wound on the right side of the photograph of the mouse shown in Figure 12-2. The composition of the conventional PRP when white blood cells (WBC) were added thereto was a white blood cell count of 180 × 10²/µl and a platelet count of 68.7 × 10⁴/µl.

(Results) - For the platelet-rich plasma with more white blood cells (conventional PRP + white blood cells (WBC)), both the length and width of the wound were smaller, demonstrating a high healing accelerating effect (Figures 12-1 and 12-2).

### Experimental Example 7

(Object) - The object of this experimental example was to examine the bactericidal and digestive capability of white blood cells included in platelet-rich plasma prepared by the conventional method (hereunder, referred to as "conventional PRP") and platelet-rich plasma obtained by the method of this invention (hereunder, referred to as "PRP of the method of this invention"). The bactericidal and digestive capability of white blood cells was measured using myeloperoxidase (MPO) as an indicator.

(Materials and Method) - For the conventional PRP, approximately 7 ml of platelet-rich plasma was prepared by employing as a starting material a substance having a total volume of 15 ml obtained by adding blood to 1.5 ml of an aqueous solution containing ACD at 3.8 w/v%, and then centrifuging for 12 minutes at 1100 rpm at room temperature to fractionate supernatant.

For the PRP of the method of this invention, approximately 7 ml of platelet-rich plasma was prepared by employing as a starting material a substance having a total volume of 15 ml obtained by adding blood to 1.5 ml of an aqueous solution containing ACD at 3.8 w/v%, adding 30 mg of sodium poly-L-glutamate salt (molecular weight 53,785) to the starting material, allowing the mixture to stand for 30 minutes, and then fractionating supernatant.

2 ml of each of the conventional PRP and the PRP of the method of this invention were employed as measurement samples. After freezing and thawing the samples 3 times, the samples were reacted with 0.167 mg/ml o-dianisidine and 0.0005 % hydrogen peroxide solution, changes in absorbance at 450 nm were measured, and quantified using a human MPO reference standard (manufactured by Sigma Chemical Co.).

(Results) - The MPO activity of the conventional PRP was 0.045 U/ml while that of the PRP of the method of this invention was 0.72 U/ml, demonstrating that the MPO activity in PRP obtained by the method of this invention was clearly higher than that of PRP prepared by the conventional method.

### Industrial Applicability

As described in the foregoing, platelet-rich plasma having high activity can be simply and conveniently provided by the method for preparing platelet-rich plasma of this invention. In particular, platelet-rich plasma obtained by the method of this invention by employing a patient's own blood components as the raw material can be applied in the medical care field as an accelerator of tissue and/or organ repair, more specifically as an additive for bone augmentation in the periphery of a dental implant, an additive for use when transplanting bone or artificial bone to a bone defect site, a wound healing accelerator, an accelerator of tissue repair after therapy or treatment for plastic and/or cosmetic purposes, a therapeutic agent for dermatosis, a therapeutic agent for cutaneous ulcers, an agent for nerve tissue repair and/or an agent for postoperative tissue repair.

## Claims

1. A method for preparing platelet-rich plasma, comprising a step of agglutinating and sedimenting red blood cells in a selective and accelerative manner from blood.

2. The method for preparing platelet-rich plasma according to claim 1, wherein the blood is whole blood obtained by blood collection.

3. The method for preparing platelet-rich plasma according to claim 1 or 2, wherein the method of agglutinating and sedimenting red blood cells in a selective and accelerative manner comprises a step of adding a water-soluble polymer compound to the blood.

4. A method for preparing platelet-rich plasma, comprising a step of adding a water-soluble polymer compound to blood.

5. The method for preparing platelet-rich plasma according to claim 3 or 4, wherein the water-soluble polymer compound is a polymer compound having a molecular weight of 1,000 - 5,000,000.

6. The method for preparing platelet-rich plasma according to claim 3 or 4, wherein the water-soluble polymer compound is added in an amount of 0.0001 - 10 w/v% with respect to a blood volume.

7. The method for preparing platelet-rich plasma according to claim 3 or 4, wherein the water-soluble polymer compound is at least one kind selected from the following compounds:
1) a polyamino acid comprising amino acids and/or pharmacologically acceptable salts of amino acids;
2) an acidic polysaccharide and/or pharmacologically acceptable salts of these; and
3) a vinyl polymer.

8. The method for preparing platelet-rich plasma according to claim 7, wherein the polyamino acid is at least one kind selected from the group consisting of polyglutamic acid, polyaspartic acid, polyhistidine and polyasparagine.

9. The method for preparing platelet-rich plasma according to the foregoing 7, wherein the amino acids and/or pharmacologically acceptable salts, which are formed polyamino acid, are selected from the group consisting of glutamic acid, aspartic acid, histidine and asparagine, or pharmacologically acceptable salts of these.

10. The method for preparing platelet-rich plasma according to claim 9, wherein at least 20% of the amino acids which the polyamino acid comprises is glutamic acid and/or aspartic acid, or pharmacologically acceptable salts thereof.

11. The method for preparing platelet-rich plasma according to claim 9 or 10, wherein the polyamino acid is an acidic polyamino acid.

12. The method for preparing platelet-rich plasma according to claim 7, wherein the acidic polysaccharide and/or pharmacologically acceptable salt thereof is at least one kind selected from the group consisting of dextran derivatives, glycosaminoglycan, cellulose derivatives, chitosan derivatives, galacturonic acid and alginic acid, or pharmacologically acceptable salts thereof.

13. The method for preparing platelet-rich plasma according to claim 7, wherein the acidic polysaccharide and/or pharmacologically acceptable salt thereof is hyaluronic acid or a pharmacologically acceptable salt thereof.

14. The method for preparing platelet-rich plasma according to claim 7, wherein the vinyl polymer is at least one kind selected from compounds including an acidic polymer or a pharmacologically acceptable salt thereof.

15. A platelet-rich plasma prepared by the method according to any one of claim 1 - 14.

16. An accelerator of tissue and/or organ repair, which comprises the platelet-rich plasma according to claim 15.

17. An accelerator of tissue and/or organ repair, an additive for bone augmentation in the periphery of a dental implant, an additive for use when transplanting bone or artificial bone to a bone defect site, a wound healing accelerator, an accelerator of tissue repair after therapy or treatment for plastic and/or cosmetic purposes, a therapeutic agent for dermatosis, a therapeutic agent for cutaneous ulcers, an agent for nerve tissue repair and/or an agent for postoperative tissue repair which comprises the platelet-rich plasma according to claim 15.

18. A therapeutic method or a treatment method for any of the following, which comprises a step of administering the platelet-rich plasma according to claim 15:
1) bone augmentation in the periphery of a dental implant;
2) dermatosis;
3) tissue repair for plastic and/or cosmetic purposes;
4) repair of a bone defect site;
5) nerve tissue repair; and
6) postoperative tissue repair.

19. A reagent or a reagent kit for preparing platelet-rich plasma that contains at least one kind of the components described below from among water-soluble polymer compounds used for preparing platelet-rich plasma by a method comprising a step of adding a water-soluble polymer compound to blood:
1) a polyamino acid comprising amino acids and/or pharmacologically acceptable salts of amino acids;
2) an acidic polysaccharide and/or pharmacologically acceptable salt thereof; and
3) a vinyl polymer.

20. An instrument for preparing platelet-rich plasma by adding at least one kind of the components of water-soluble polymer compounds described below to blood:
1) a polyamino acid comprising amino acids and/or pharmacologically acceptable salts of amino acids;
2) an acidic polysaccharide and/or pharmacologically acceptable salt thereof; and
3) a vinyl polymer.

21. A kit for preparing platelet-rich plasma, which comprises the reagent or the reagent kit according to claim 19 and the instrument according to claim 20.
